(19) Europäisches Patentamt
European Patent Office
Office européen des brevets

(11) Veröffentlichungsnummer: **0 341 475 B1**

# (12) EUROPÄISCHE PATENTSCHRIFT

(45) Veröffentlichungstag der Patentschrift: **29.09.93**

(21) Anmeldenummer: **89107326.4**

(22) Anmeldetag: **24.04.89**

Die Akte enthält technische Angaben, die nach dem Eingang der Anmeldung eingereicht wurden und die nicht in dieser Patentschrift enthalten sind.

(51) Int. Cl.$^5$: **C07C 233/57**, C07C 231/02, C07C 61/15, C07C 69/635, A01N 53/00

## (54) Stereoisomere von N-(R)-1-Aryl-ethyl)-1-alkyl-2,2-dichlo-cyclopropancarbonsäureamiden.

(30) Priorität: **07.05.88 DE 3815728**

(43) Veröffentlichungstag der Anmeldung:
**15.11.89 Patentblatt 89/46**

(45) Bekanntmachung des Hinweises auf die Patenterteilung:
**29.09.93 Patentblatt 93/39**

(84) Benannte Vertragsstaaten:
**CH DE FR GB IT LI**

(56) Entgegenhaltungen:
**EP-A- 0 170 842**
**EP-A- 0 257 448**
**US-A- 3 856 976**

**CHEMICAL ABSTRACTS, Band 107, Nr. 23, 7. Dezember 1987, Seite 221, Zusammenfassung Nr. 213585j, Columbus, Ohio, US; & JP-A-62 161 707**

(73) Patentinhaber: **BAYER AG**
**D-51368 Leverkusen(DE)**

(72) Erfinder: **Kraatz, Udo, Dr.**
**Andreasstrasse 22a**
**D-5090 Leverkusen 1(DE)**
Erfinder: **Hänssler, Gerd, Dr.**
**Am Arenzberg 50a**
**D-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Stereoisomere von N-(R)-(1-Aryl-ethyl)-1-alkyl-2,2-dichlor-cyclopropancarbonsäureamiden, ein Verfahren zu ihrer Herstellung und ihre Verwendung zur Bekämpfung von Pilzen.

Es ist bereits bekannt, daß Isomerengemische von bestimmten Cyclopropancarbonsäureamiden, wie z. B. von N-(1-(4-Chlor-phenyl)-ethyl)-2,2-dichlor-1-ethyl-3-methylcyclopropancarbonsäureamid fungizide Eigenschaften aufweisen (vgl. EP-A 170 842 / US-P 4 710 518). Die Wirkung der bekannten Verbindungen ist jedoch nicht in allen Belangen zufriedenstellend.

Auch bestimmte optisch aktive Formen von substituierten Cyclopropancarbonsäureamiden sind bereits bekannt geworden (vgl. EP-A 257 448).

Es wurden nun neue Stereoisomere von N-(R)-(1-Arylethyl)-1-alkyl-2,2-dichlor-cyclopropancarbonsäureamiden der Formeln (Ia) und (Ib)

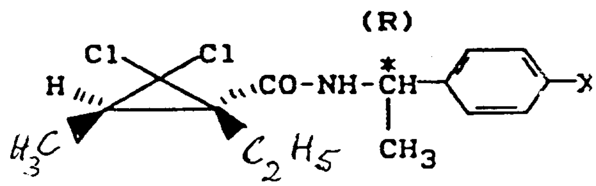

(Ia)

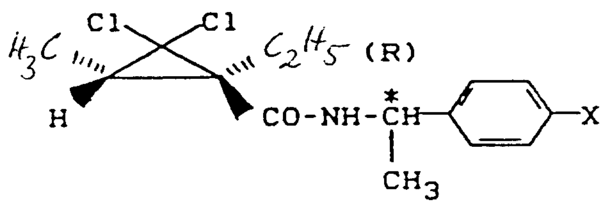

(Ib)

in welchen

X für Wasserstoff oder Chlor steht,

gefunden.

Die Erfindung betrifft sowohl die einzelnen Stereoisomeren der Formeln (Ia) und (Ib) als auch deren Gemische.

Man erhält die neuen Verbindungen der Formeln (Ia) und (Ib), wenn man

- zur Herstellung ihrer Gemische in einer ersten Stufe -

Gemische aus (1R)- und (1S)-1-Alkyl-2,2-dichlor-cyclopropancarbonsäurechloriden der Formeln (IIa) und (IIb)

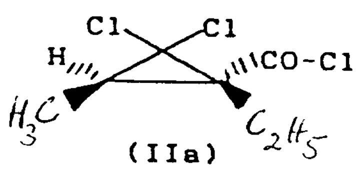

(IIa)

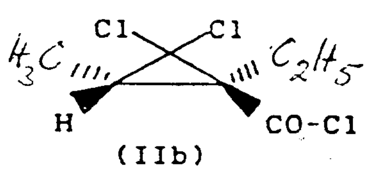

(IIb)

mit (R)-1-Aryl-ethylaminen der Formel (III)

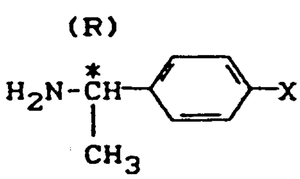

(III)

in welcher

X die oben angegebene Bedeutung hat,

gegebenenfalls in Gegenwart eines Säureakzeptors und gegebenenfalls in Gegenwart eines Verdünnungsmittels umsetzt und wenn man gegebenenfalls

- zur Herstellung der einzelnen Stereoisomeren der Formeln (Ia) und (Ib) -

die erhaltenen Diastereomerengemische aus den Verbindungen der Formeln (Ia) und (Ib) in einer zweiten Stufe aufgrund der unterschiedlichen physikalischen Eigenschaften nach üblichen Methoden trennt.

Die neuen Stereoisomeren von N-(R)-(1-Aryl-ethyl)-1-alkyl-2,2-dichlor-cyclopropancarbonsäureamiden der Formeln (Ia) und (Ib) sowie deren Gemische zeichnen sich durch starke Wirkung gegen Pilze aus.

Überraschenderweise zeigen die neuen Stereoisomeren der Formeln (Ia) und (Ib), insbesondere diejenigen der Formel (Ia) wie auch die Gemische der Verbindungen der Formeln (Ia) und (Ib) u.a. eine erheblich stärkere fungizide Wirkung als das bekannte Isomerengemisch von N-(1-(4-Chlor-phenyl)-ethyl)-2,2-dichlor-1-ethyl-3-methylcyclopropancarbonsäureamid.

Die Erfindung betrifft vorzugsweise die Stereoisomeren der Formel (Ia) sowie Gemische aus den Stereoisomeren der Formeln (Ia) und (Ib), welche wenigstens 50 % der Stereoisomeren der Formel (Ia) enthalten.

Verwendet man ein 1 : 1-Gemisch aus (1R)- und (1S)-2,2-Dichlor-1-ethyl-3t-methyl-1r-cyclopropancarbonsäurechlorid (Nomenklatur vgl. Beilstein) und (R)-1-Phenylethylamin als Ausgangsstoffe, so kann der Reaktionsablauf bei der ersten Stufe des erfindungsgemäßen Verfahrens durch das folgende Formelschema wiedergegeben werden:

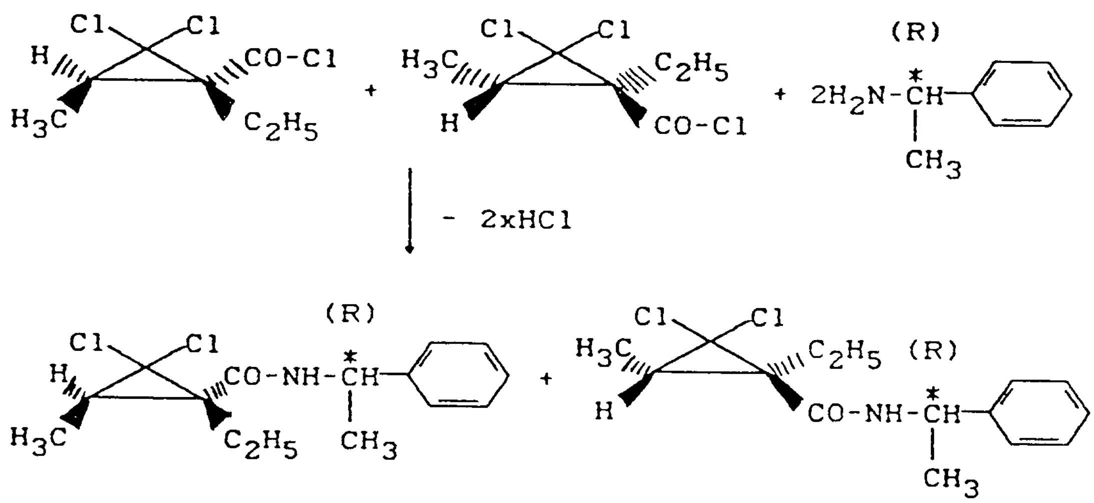


Die beim erfindungsgemäßen Verfahren zur Herstellung von Verbindungen der Formel (Ia) und (Ib) als Ausgangsstoffe zu verwendenden 1-Alkyl-2,2-dichlor-cyclopropancarbonsäurechloride sind durch die Formeln (IIa) und (IIb) definiert. Es handelt sich um die Verbindungen (1R)- und (1S)-2,2-Dichlor-1-ethyl-3t-methyl-1r-cyclopropancarbonsäurechlorid.

Man erhält 1 : 1-Gemische von Verbindungen der Formeln (IIa) und (IIb), wenn man entsprechende Gemische von (1R)- und (1S)-1-Alkyl-2,2-dichlor-cyclopropancarbonsäuren der Formeln (IIIa) und (IIIb)

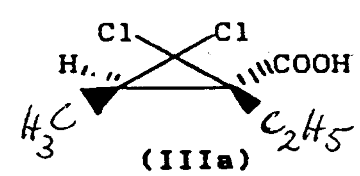
(IIIa)

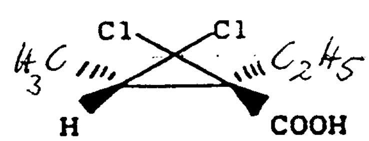
(IIIb)

mit einem Chlorierungsmittel, wie z. B. Thionylchlorid, gegebenenfalls in Gegenwart eines Katalysators, wie z. B. Dimethylformamid, und gegebenenfalls in Gegenwart eines Verdünnungsmittels, wie z. B. Methylenchlorid, bei Temperaturen zwischen 10 °C und 50 °C umsetzt.

Die als Ausgangsstoffe benötigten 1-Alkyl-2,2-dichlorcyclopropancarbonsäuren sind durch die Formeln (IIIa) und (IIIb) definiert. Es handelt sich um die Verbindungen (1R)- und (1S)-2,2-Dichlor-1-ethyl-3t-methyl-1r-cyclopropancarbonsäure.

Man erhält 1 : 1-Gemische von Verbindungen der Formeln (IIIa) und (IIIb), wenn man entsprechende Gemische von (1R)- und (1S)-1-Alkyl-2,2-dichlor-cyclopropancarbonsäureestern der Formeln (IVa) und (IVb)

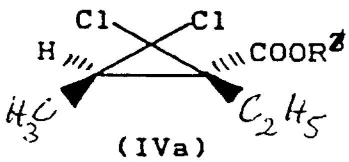

(IVa)

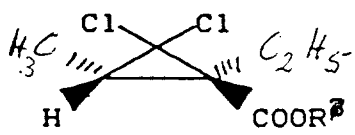

(IVb)

in welchen

R für Alkyl, vorzugsweise für Methyl oder Ethyl, steht,

mit einer wäßrigen Alkalilauge, wie z. B. Natronlauge, gegebenenfalls in Gegenwart eines organischen Lösungsmittels, wie z. B. Methanol oder Ethanol, bei Temperaturen zwischen 20 °C und 100 °C umsetzt und dann mit einer starken Säure, wie z. B. Salzsäure, ansäuert.

Die als Ausgangsstoffe benötigten 1-Alkyl-2,2-dichlorcyclopropancarbonsäureester sind durch die Formeln (IVa) und (IVb) allgemein definiert.

Als Beispiele für die Ausgangsstoffe der Formeln (IVa) und (IVb) seien genannt:
(1R)- und (1S)-2,2-Dichlor-1-ethyl-3t-methyl-1r-cyclopropancarbonsäure-methylester und -ethylester.

Man erhält 1 : 1-Gemische von Verbindungen der Formeln (IVa) und (IVb), wenn man Alkencarbonsäureester der Formel (V)

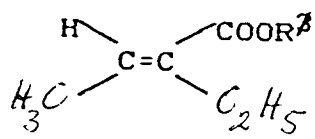

(V)

in welcher

R die oben angegebene Bedeutung hat,

mit Dichlorcarben, welches man gegebenenfalls in situ, beispielsweise aus Chloroform und einer starken Base, wie z. B. Natriumhydroxid oder Kaliumhydroxid, erzeugt, gegebenenfalls in Gegenwart von Verdünnungsmitteln, wie z. B. Chloroform und Wasser, und gegebenenfalls in Gegenwart eines Phasentransfer-Katalysators, wie z. B. Tetrabutylammoniumbromid, bei Temperaturen zwischen 10 °C und 60 °C umsetzt.

Die Ausgangsstoffe der Formel (V) sind bereits bekannt (vgl. J. Organomet. Chem. 96 (1975), 163 - 168).

Die beim erfindungsgemäßen Verfahren zur Herstellung von Verbindungen der Formeln (Ia) und (Ib) weiter als Ausgangsstoffe zu verwendenden (R)-1-Aryl-ethylamine sind durch die Formel (III) definiert.

Es handelt sich um (R)-1-Phenyl-ethylamin und (R)-1-(4-Chlor-phenyl)-ethylamin.

Die Ausgangsstoffe der Formel (777) sind bereits bekannt (vgl. Indian J. Chem. 13 (1975), 631).

Die erste Stufe des erfindungsgemäßen Verfahrens zur Herstellung der neuen Stereoisomeren der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können in der ersten Stufe des erfindungsgemäßen Verfahrens alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können in der ersten Stufe des erfindungsgemäßen Verfahrens in einem größeren Bereich variiert werden. Im allgemeinen arbeitet man bei Temperaturen zwischen -20 °C und +80 °C, vorzugsweise bei Temperaturen zwischen 0 °C und 50 °C.

Das erfindungsgemäße Verfahren wird in der ersten Stufe im allgemeinen bei Normaldruck durchgeführt.

Zur Durchführung der ersten Stufe des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt nach der ersten Stufe des erfindungsgemäßen Verfahrens nach üblichen Methoden.

Im allgemeinen geht man so vor, daß man das Reaktionsgemisch - gegebenenfalls nach Einengen - mit Wasser und einem mit Wasser praktisch nicht mischbaren organischen Lösungsmittel, wie z. B. Methylenchlorid, schüttelt, die organische Phase mit einer wäßrigen Mineralsäure, wie z. B. Salzsäure, wäscht, trocknet, filtriert, das Filtrat einengt, den Rückstand durch Verreiben mit einem geeigneten Lösungsmittel, wie z. B. Cyclohexan, zur Kristallisation bringt und absaugt.

In der zweiten Stufe kann die Trennung der Diastereomeren (Ia) und (Ib) nach den für derartige Zwecke geeigneten Methoden vorgenommen werden, also z. B, durch fraktionierte Kristallisation oder auch mit Hilfe von chromatographischen Verfahren.

Besonders bevorzugt wendet man säulenchromatographische Trennverfahren an, wie z. B. die Hochdruckfiltration über eine Kieselgelsäule mit einem Elutionsgemisch aus Hexan, Cyclohexan, Methyl-tert-butylether, Heptan, Tetrachlorkohlenstoff und/oder Propionitril.

Die erfindungsgemäßen Wirkstoffe weisen eine starke biologische Wirkung auf und können zur Bekämpfung von Pilzen als Fungizide praktisch eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;

Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich insbesondere zur Bekämpfung von Pyricularia oryzae an Reis.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

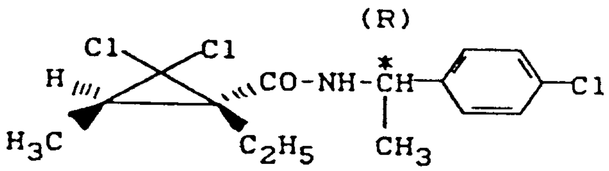

(1a)

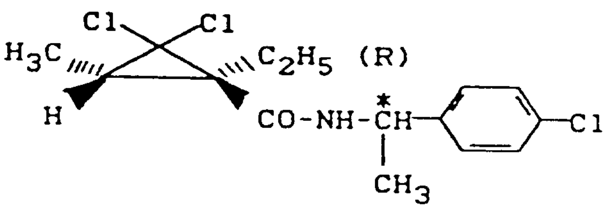

(1b)

9,5 g (44 mmol) einer 1 : 1-Mischung aus (1R)- und (1S)-2,2-Dichlor-1-ethyl-3t-methyl-1r-cyclopropancarbonsäurechlorid werden unter Rühren und Eiskühlung zu einer Mischung aus 6,2 g (40 mmol) (R)-1-(4-Chlor-phenyl)-ethylamin, 4,8 g (48 mmol) Triethylamin und 40 ml Methylenchlorid gegeben. Das Reaktionsgemisch wird dann 4 Stunden bei 20 °C gerührt, mit Wasser und Methylenchlorid verdünnt und geschüttelt. Die organische Phase wird abgetrennt, mit 2N-Salzsäure gewaschen, mit Magnesiumsulfat getrocknet und filtriert. Vom Filtrat wird das Lösungsmittel im Wasserstrahlvakuum abdestilliert, das im Rückstand verbliebene Produkt durch Verreiben mit Cyclohexan zur Kristallisation gebracht und durch Absaugen isoliert.

Man erhält 13,8 g (98 % der Theorie) eines Diastereomerengemisches (1a/1b) von N-(R)-(1-(4-Chlor-phenyl)-ethyl)-2,2-dichlor-1-ethyl-3t-methyl-1r-cyclopropancar bonsäureamid vom Schmelzpunkt 144 °C - 146 °C.
$[\alpha]_D^{25}$ : + 40,92 ($CHCl_3$, c = 5,1).

10 g (30 mmol) des Diastereomerengemisches werden mittels HPLC (high performance liquid chromatography / hochauflösende Flüssigkeitschromatographie) an einer Kieselgelsäule (Merck, 5 - 20 µ Korngröße) mit dem Elutionsmittelgemisch Heptan/Methyl-tert-butylether (85 : 15) getrennt.

Als erste Fraktion werden 4,1 g N-(R)-(1-(4-Chlor-phenyl)-ethyl)-(1R)-2,2-dichlor-1-ethyl-3t-methyl-1r-cyclopropancarbonsäureamid (1b) vom Schmelzpunkt 158 °C eluiert.
$[\alpha]_D^{25}$ : + 20,05 ($CHCl_3$, c = 2,00).

Als zweite Fraktion erhält man 4,4 g N-(R)-(1-(4-Chlorphenyl)-ethyl)-(1S)-2,2-dichlor-1-ethyl-3t-methyl-1r-cyclopropancarbonsäureamid (1a) vom Schmelzpunkt 154 °C.
$[\alpha]_D^{25}$ : + 61,01 ($CHCl_3$, c = 2,03).

Analog Beispiel 1 können die nachstehend aufgeführten Verbindungen erhalten werden.

Beispiel 2

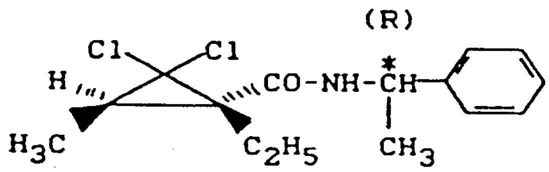

+

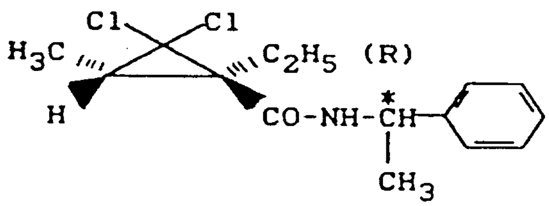

$[\alpha]_D^{25}$ : + 40,49 ($CHCl_3$)

Schmelzpunkt: 120 °C

Ausgangsstoffe der Formeln (IIa) und (IIb)

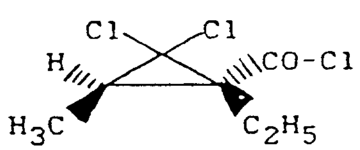

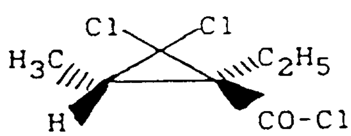

29 g (0,15 Mol) einer 1 : 1-Mischung aus (1R)- und (1S)-2,2-Dichlor-1-ethyl-3t-methyl-1r-cyclopropancarbonsäure (IIIa/IIIb) werden in 30 ml Methylenchlorid gelöst. Es werden dann 2 Tropfen Dimethylformamid und anschließend unter Rühren 28,6 g (0,24 Mol) Thionylchlorid tropfenweise dazu gegeben und das Gemisch wird noch 30 Minuten unter Rückfluß zum Sieden erhitzt. Dann wird durch Destillation im Wasserstrahlvakuum aufgearbeitet.

Man erhält 25 g (78 % der Theorie) einer 1 : 1-Mischung aus (1R)- und (1S)-2,2-Dichlor-1-ethyl-3t-methyl-1r-cyclopropancarbonsäurechlorid vom Siedepunkt 80 °C - 85 °C (15 Torr/2 kPa).

Ausgangsstoffe der Formeln (IIIa) und (IIIb)

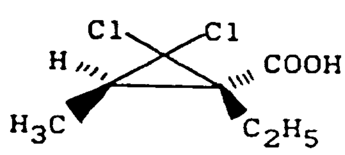

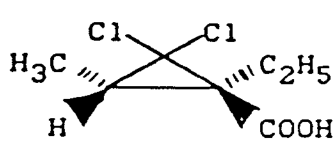

Eine Lösung von 67 g (1,675 Mol) Natriumhydroxid in 80 ml Wasser wird zu einer Lösung von 185 g (0,82 Mol) einer 1 : 1-Mischung von (1R)- und (1S)-2,2-Dichlor-1-ethyl-3t-methyl-1r-cyclopropancarbonsäureethylester in 400 ml Ethanol gegeben und das Reaktionsgemisch wird 3 Stunden bei 80 °C gerührt. Anschließend wird das Ethanol im Wasserstrahlvakuum weitgehend abdestilliert, der Rückstand mit Wasser verdünnt und mit 10 %iger Salzsäure angesäuert. Dann wird mit Methylenchlorid extrahiert, die organische Phase eingeengt, der Rückstand durch Verreiben mit Petrolether zur Kristallisation gebracht und das Produkt durch Absaugen isoliert.

Man erhält 108 g (67 % der Theorie) einer 1 : 1-Mischung aus (1R)- und (1S)-2,2-Dichlor-1-ethyl-3t-methyl-1r-cyclopropancarbonsäure vom Schmelzpunkt 77 °C - 79 °C.

Ausgangsstoffe der Formeln (IVa) und (IVb)

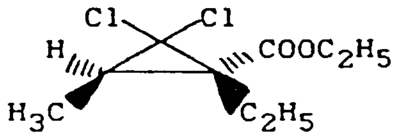

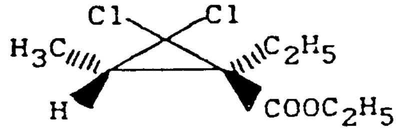

Eine Lösung von 405 g (6,4 Mol) 88 %igem Kaliumhydroxid in 307 ml Wasser sowie 8,3 g (0,026 Mol) Tetrabutylammoniumbromid werden zu einer Lösung von 181 g (1,28 Mol) *E-2-Ethyl-2-butensäureethylester in 550 ml Chloroform gegeben und das Reaktionsgemisch wird 20 Stunden bei 40 °C intensiv gerührt. Die organische Phase wird dann abgetrennt, mit 1N-Salzsäure gewaschen und im Wasserstrahlvakuum destilliert.

Man erhält 185 g (64 % der Theorie) einer 1 : 1-Mischung aus (1R)- und (1S)-2,2-Dichlor-1-ethyl-3t-methyl-1r-cyclopropancarbonsäureethylester vom Siedepunkt 95 °C - 102 °C (15 Torr/2kPa).

Ausgangsstoffe der Formel (III)

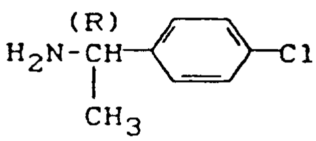

Die auf ca. 60 °C - 70 °C erwärmten Lösungen von 91 g (0,587 Mol) rac. p-Chlorphenethylamin in 300 ml Ethanol und 122 g (S)-(-)-N-Phenylcarbamatmilchsäure in 800 ml Ethanol werden vereinigt und über Nacht stehen gelassen. Der Kristallbrei wird abgesaugt (93 g) und mit 200 ml 10 %iger Natronlauge/ 300 ml Methylenchlorid behandelt. Die organische Phase wird mit Wasser gewaschen, über Magnesiumsulfat getrocknet und einrotiert.

Man erhält 44,5 g (+)-Enantiomeres mit einer optischen Reinheit von 89,4 % e.e. (enantiomeric excess).

44 g (0,28 Mol) dieses Amins werden in 150 ml Ethanol gelöst, auf 60 °C erwärmt und mit einer auf ebenfalls 60 °C erwärmten Lösung von 59 g (0,28 Mol) (S)-(-)-N-Phenylcarbamatmilchsäure in 400 ml Ethanol vereinigt. Das langsam auskristallisierende Salz wird nach Stehen über Nacht abgesaugt (74 g) und wie vorher beschrieben, mit 300 ml Methylenchlorid/ 10 %iger Natronlauge wieder zerlegt.

Nach dem Waschen, Trocknen und Einengen der organischen Phase erhält man 40,4 g (88,8 % der Theorie) (+)-Enantiomeres mit einer optischen Reinheit von 97,4 % e.e. (enantiomeric excess).

Verwendungsbeispiel

Pyricularia-Test (Reis) /protektiv

Lösungsmittel: 12,5 Gewichtsteile Aceton
Emulgator: 0,3 Gewichtsteile Alkylarylpolyglykolether
Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

*Nomenklatur (E/Z) vg. J. Am. Chem. Soc. 90 (1968), 509-510.

Die erfindungsgemäße Verbindung der Formel (1a) zeigt eine deutliche Überlegenheit in der Wirksamkeit gegenüber dem Stand der Technik in diesem Test.

Tabelle

Pyricularia-Test (Reis)/protektiv

| Wirkstoff | Wirkstoffkonzentration in % | Wirkungsgrad in % der unbehandelten Kontrolle |
|---|---|---|
| 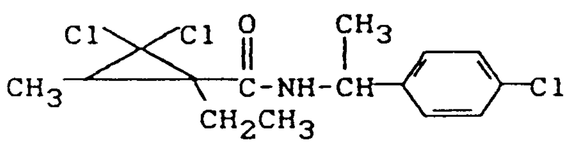 (Bekannt) | 0,0025 | 30 |
| 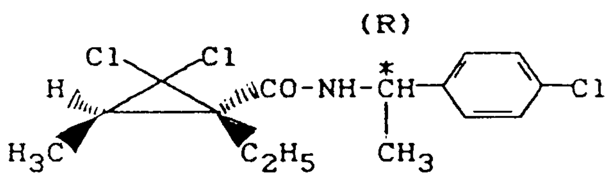 (1a) | 0,0025 | 89 |

**Patentansprüche**

1. Stereoisomere von N-(R)-(1-Aryl-ethyl)-1-alkyl-2,2-dichlor-cyclopropancarbonsäureamiden der Formeln (Ia) und (Ib)

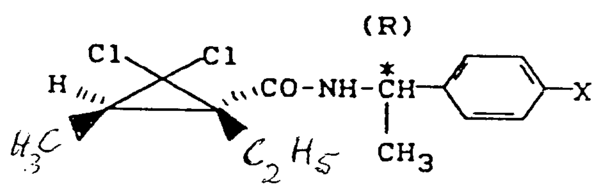 (Ia)

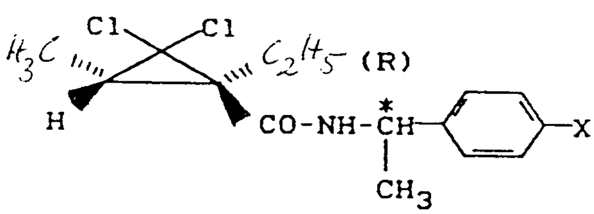 (Ib)

und deren Gemische,
in welchen
X für Wasserstoff oder Chlor steht.

2. Gemische von Stereoisomeren von N-(R)-(1-Arylethyl)-1-alkyl-2,2-dichlor-cyclopropancarbonsäureamiden gemäß Anspruch 1, welche wenigstens 50 % der Stereoisomeren der Formel (Ia) enthalten.

3. Verfahren zur Herstellung von Gemischen der Stereoisomeren von N-(R)-(1-Aryl-ethyl)-1-alkyl-2,2-dichlor-cyclopropancarbonsäureamiden und den reinen Isomeren der Formeln (Ia) und (Ib) gemäß Anspruch 1, dadurch gekennzeichnet, daß man Gemische aus (1R)- und (1S)-1-Alkyl-2,2-dichlor-cyclopropancarbonsäurechloriden der Formeln (IIa) und (IIb)

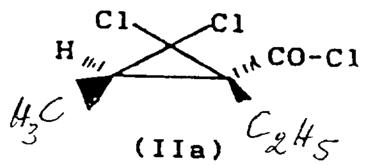
(IIa)

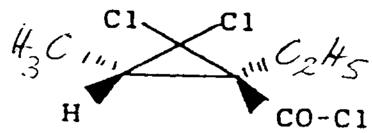
(IIb)

mit (R)-1-Aryl-ethylaminen der Formel (III)

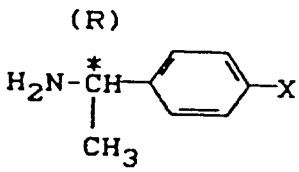
(III)

in welcher

X die oben angegebene Bedeutung hat,

gegebenenfalls in Gegenwart eines Säureakzeptors und gegebenenfalls in Gegenwart eines Verdünnungsmittels umsetzt und gegebenenfalls zur Herstellung der einzelnen Stereoisomeren der Formeln (Ia) und (Ib) die erhaltenen Diastereomerengemische aus den Verbindungen der Formeln (Ia) und (Ib) in einer zweiten Stufe aufgrund der unterschiedlichen physikalischen Eigenschaften nach üblichen Methoden trennt.

4. Fungizide Mittel, gekennzeichnet durch einen Gehalt an mindestens einem Stereoisomeren oder einem Gemisch der Stereoisomeren der Formeln (Ia) und (Ib) nach Anspruch 1.

5. Verwendung von einem Stereoisomeren oder einem Gemisch der Stereoisomeren der Formeln (Ia) und (Ib) nach Anspruch 1 zur Bekämpfung von Pilzen.

6. Verfahren zur Bekämpfung von Pilzen, dadurch gekennzeichnet, daß man ein Stereoisomeres oder ein Gemisch der Stereoisomeren der Formeln (Ia) und (Ib) nach Anspruch 1 auf Pilze und/oder ihren Lebensraum einwirken läßt.

7. Verfahren zur Herstellung von fungiziden Mitteln, dadurch gekennzeichnet, daß man ein Stereoisomeres oder ein Gemisch der Stereoisomeren der Formeln (Ia) und (Ib) nach Anspruch 1 mit Streckmitteln und/oder oberflächenaktiven Mitteln vermischt.

## Claims

1. Stereoisomers of N-(R)-(1-aryl-ethyl)-1-alkyl-2,2-dichloro-cyclopropanecarboxamides of the formulae (Ia) and (Ib)

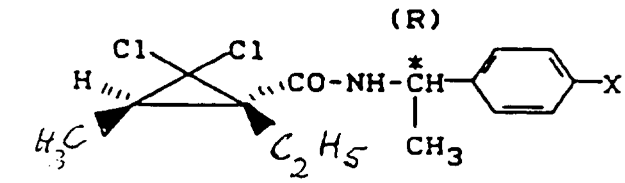

(Ia)

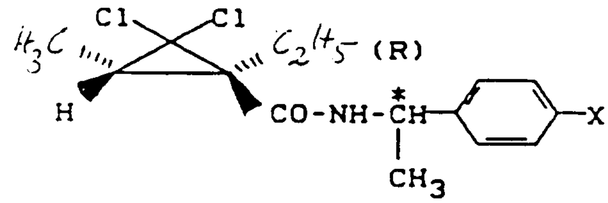

(Ib)

and their mixtures
in which
X represents hydrogen or chlorine.

2. Mixtures of stereoisomers of N-(R)-(1-aryl-ethyl)-1-alkyl-2,2-dichloro-cyclopropanecarboxamides according to Claim 1 which contain at least 50% of the stereoisomers of the formula (Ia).

3. Process for the preparation of mixtures of the stereoisomers of N-(R)-(1-aryl-ethyl)-1-alkyl-2,2-dichloro-cyclopropanecarboxamides and the pure isomers of the formulae (Ia) and (Ib) according to Claim 1, characterised in that mixtures of (1R)- and (1S)-1-alkyl-2,2-dichloro-cyclopropanecarbonyl chlorides of the formulae (IIa) and (IIb)

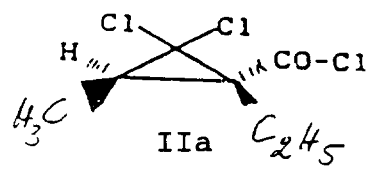

IIa

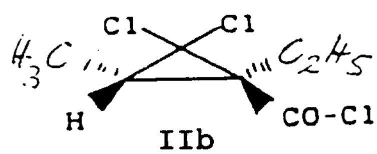

IIb

are reacted with (R)-1-aryl-ethylamines of the formula (III)

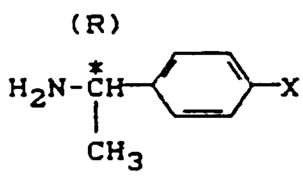

(III)

in which
X has the abovementioned meaning,
if appropriate in the presence of an acid acceptor and if appropriate in the presence of a diluent, and, if appropriate, the resulting mixtures of diastereomers of the compounds of the formulae (Ia) and (Ib) are separated in a second step by customary methods on the basis of the different physical properties, in order to prepare the individual stereoisomers of the formulae (Ia) and (Ib).

4. Fungicidal agents, characterized in that they contain at least one stereoisomer or a mixture of the stereoisomers of the formulae (Ia) and (Ib) according to Claim 1.

5. Use of one stereoisomer or a mixture of the stereoisomers of the formulae (Ia) and (Ib) according to Claim 1 for combating fungi.

6. Method of combating fungi, characterized in that one stereoisomer or a mixture of the stereoisomers of the formulae (Ia) and (Ib) according to Claim 1 is allowed to act on fungi and/or their environment.

7. Process for the preparation of fungicidal agents, characterized in that one stereoisomer or a mixture of the stereoisomers of the formulae (Ia) and (Ib) according to Claim 1 is mixed with extenders and/or surface-active agents.

**Revendications**

1. Stéréo-isomères de N-(R)-(1-aryléthyl)-1-alkyl-2,2-dichloro-cyclopropanecarboxamides de formules (Ia) et (Ib)

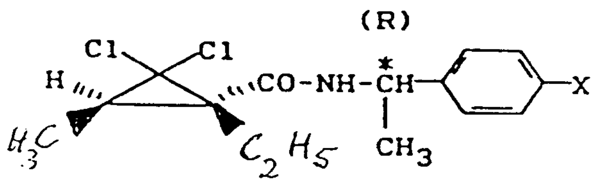

(Ia)

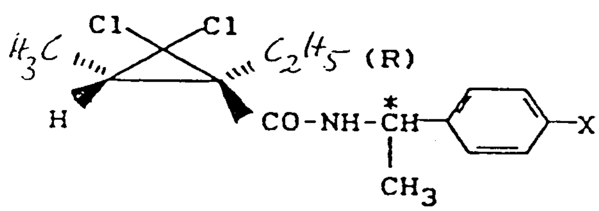

(Ib)

et leurs mélanges,
formules dans lesquelles
X représente l'hydrogène ou le chlore.

2. Mélanges de stéréo-isomères de N-(R)-(1-aryléthyl)-1-alkyl-2,2-dichloro-cyclopropanecarboxamides suivant la revendication 1, qui contiennent au moins 50 % des stéréo-isomères de formule (Ia).

3. Procédé de préparation de mélanges des stéréo-isomères de N-(R)-(1-aryléthyl)-1-alkyl-2,2-dichloro-cyclopropanecarboxamideset des isomères purs de formules (Ia) et (Ib) suivant la revendication 1, caractérisé en ce qu'on fait réagir des mélanges de chlorures d'acides (1R)- et (1S)-1-alkyl-2,2-dichloro-cyclopropanecarboxyliques de formules (IIa) et (IIb)

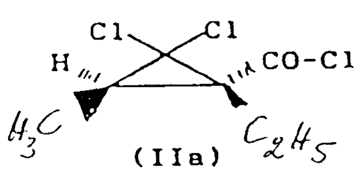

(IIa)

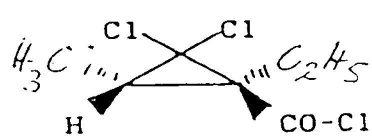

(IIb)

avec des (R)-1-aryléthylamines de formule (III)

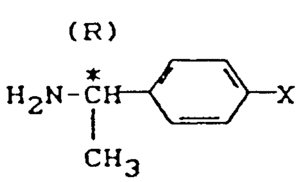

(III)

dans laquelle

X a la définition indiquée ci-dessus,

le cas échéant en présence d'un accepteur d'acide et en la présence éventuelle d'un diluant et pour la préparation éventuelle des stéréo-isomères individuels de formules (Ia) et (Ib), on sépare par des opérations connues les mélanges de diastéréo-isomères obtenus constitués des composés de formules (Ia) et (Ib) dans une seconde étape sur la base des différences de propriétés physiques.

**4.** Compositions fongicides, caractérisées par une teneur en au moins un stéréo-isomère ou un mélange de stéréo-isomères de formules (Ia) et (Ib) suivant la revendication 1.

**5.** Utilisation d'un stéréo-isomère ou d'un mélange de stéréo-isomères de formules (Ia) et (Ib) suivant la revendication 1 pour combattre des champignons.

**6.** Procédé pour combattre des champignons, caractérisé en ce qu'on fait agir un stéréo-isomère ou un mélange de stéréo-isomères de formules (Ia) et (Ib) suivant la revendication 1 sur des champignons et/ou sur leur milieu.

**7.** Procédé de préparation de compositions fongicides, caractérisé en ce qu'on mélange un stéréo-isomère ou un mélange de stéréo-isomères de formules (Ia) et (Ib) suivant la revendication 1 avec des diluants et/ou des agents tensioactifs.